# EUROPEAN PATENT SPECIFICATION

(11) **EP 1 514 553 B1**
(45) Date of publication and mention of the grant of the patent: **27.02.2008**
(21) Application number: 03255553.4
(22) Date of filing: 05.09.2003
(51) Int. Cl.: A61K 35/74, C12N 1/20

(54) **Lactic acid bacteria powder double-coated using protein and polysaccharide and method preparing the same and a dosage form thereof**
Zweifach Coating-beschichteten Milchsäurenbakterienpulver mit Protein und Polysaccharid und das Verfahren zur deren Herstellung sowie deren Dosisform
Poudre de bactéries lactiques doublement envelopée de protéine et de polysaccharide et le procédé pour leur préparation ainsi que leur forme galénique

(43) Date of publication of application: 16.03.2005
(73) Proprietor: Chung, Myung-jun, Seocho-gu, Seoul 130-070 (KR)
(72) Inventor: Chung, Myung-jun, Seocho-gu Seoul 130-070 (KR); Cho, Young-jai, Kimpo-shi Kyunggi-do 415-748 (KR); Kim, Soo-dong, Youngdeoungpo-gu Seoul 150-050 (KR); Hong, Un-pyoe, Kimpo-shi Kyunggi-do 415-864 (KR)
(74) Representative: Thomson, Craig Richard

(56) References cited:
- WO-A-99/20745
- GB-A- 1 190 386
- US-A- 3 677 897
- US-A- 4 888 171
- US-B1- 6 455 052
- DATABASE WPI Derwent Publications Ltd., London, GB; AN 2003-338917 XP002268468 CHUNG M J: "Mabufacture method protein coating-lactic acid bacteria" & KR 2002 069 862 A (CHUN-I), 5 September 2002 (2002-09-05)

## Description

### BACKGROUND OF THE INVENTION

The present invention relates to a lactic acid bacteria(LAB) powder double-coated using a protein and a polysaccharide, a method for preparing the LAB powder, and a method for manufacturing a dosage form containing the LAB powder. More particularly, the present invention relates to a double-coated LAB powder with a high survival rate of LAB cell in the body by doubly coating LAB with a protein and a polysaccharide, a method for preparing the LAB powder, and a method for manufacturing a dosage form containing the LAB powder which can minimize the death of the viable cell during manufacture of the dosage form.

### Description of the Related Art

Generally, LAB reside in the human intestine and exhibit a variety of physiological effects including activation of intestinal motility (peristalsis), growth inhibition of harmful bacteria, promotion of vitamins and immunostimulatory material formation, etc. In view of the structure of the human body, since ingested LAB reach the intestine through the stomach, they are killed by gastric acid secreted in the stomach and thus the physiological activities of LAB species cannot be sufficiently exerted.

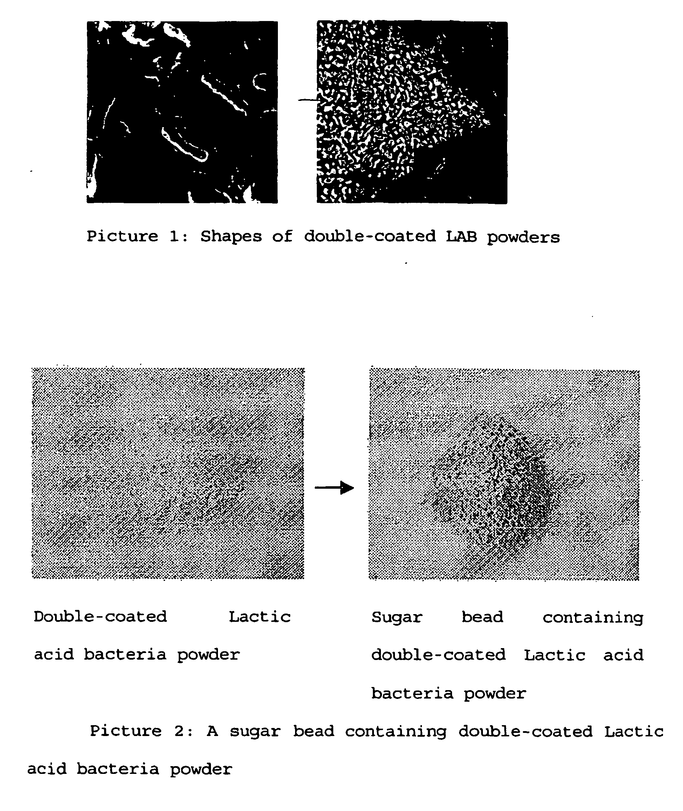

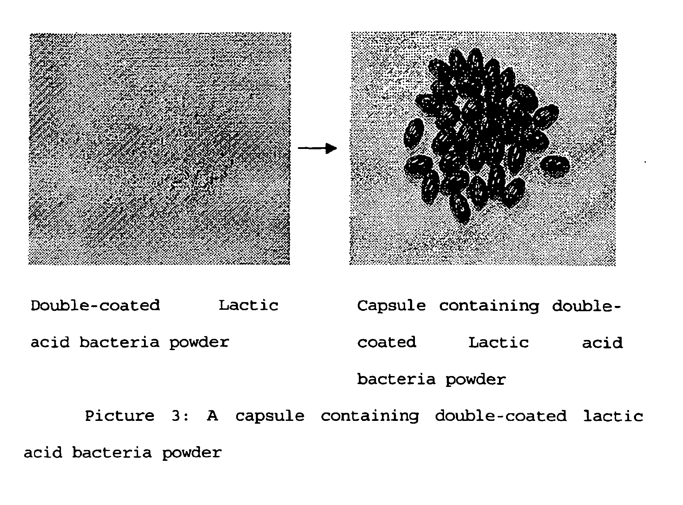

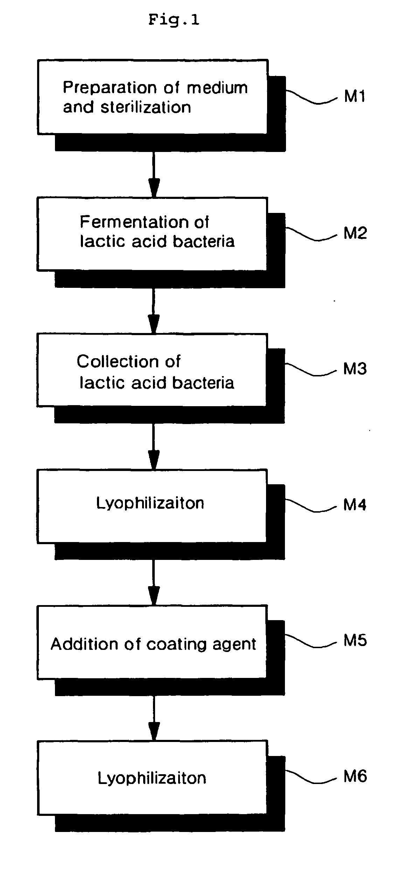

A conventional method for preparing an uncoated LAB powder comprises the steps of fermentation of LAB, collection of the LAB cells and lyophilization (see Fig. 1). Since the uncoated LAB powder is highly sensitive to air, moisture and temperature, serious problems, such as instability during storage and distribution and poor processability during manufacture of consumer products, are encountered. As described above, since most of the LAB ingested by a person or animal are killed by direct contact with gastric acid and bile acid, they cannot be stably resided in the intestine and useful efficiency and effects of LAB cannot be sufficiently assured.

To solve these problems, LAB has been coated. Conventional methods for coating LAB include the use of enteric coating agents and microencapsulation using gelatin, saccharides, gums, etc. These coating methods further comprise an additional step of adding a coating agent following collecting LAB.

The coating of LAB is commonly carried out by adding a coating agent to a freeze-dried LAB powder. The general procedure is shown in Fig. 1.

A first step (M1, M2) for culturing LAB is carried out by culturing LAB in a fermentation medium supplemented with peptones, beef extract, yeast extract, glucose and inorganic ions in an anaerobic apparatus. These medium ingredients are mainly composed of water-soluble ones required for the proliferation of LAB. Next, the LAB culture is collected using a centrifugation or ultrafiltration to separate the LAB culture and filtrate and to concentrate the LAB culture.

A step of rapidly freezing and lyophilizing the LAB culture (M4) causes the death of the LAB cells. For this reason, a cryoprotectant including saccharides and amino acids is added to dry and powder the LAB culture, prior to lyophilizing. Then, a coating agent in an aqueous solution is added to the freeze-dried LAB powder (M5). The resulting mixture is stirred and re-lyophilized (M6).

Alternatively, the coating step may be carried out using a microencapsulation process which comprises the steps of adding a coating agent capable of forming ultrafine, spherical beads and spraying the mixture using a nozzle.

As noted above, since the conventional methods for coating LAB comprise the steps of mixing and stirring a coating agent, or using a microencapsulation process, following collecting a LAB culture and dry-powdering the LAB culture, they are economically disadvantageous in terms of costly coating agent and additional steps. In addition, since there is a risk of microorganism contamination, aseptic process is difficult. Furthermore, since a cryoprotectant and a stabilizer are required to assure the survival rate and stability of LAB during the lyophilizing step, the methods involve excessive processing steps and consumption of materials.

Based on these problems of the prior arts, there is a need to develop coated LAB powders which can effectively control the reaction with air and moisture, exhibit excellent resistance to heat, acid and bile, and assure viability, efficiency and effects of LAB. In addition, there is a need to develop methods for aseptically preparing the powders with low-cost materials and low manufacturing costs. Furthermore, when a coating agent selected from proteins, polysaccharides, gums, various enteric coating materials, etc., is used alone, coating effect of the coating agent is unsatisfactory, which affects the stability of LAB. Accordingly, it is necessary to maximize the coating effect by combining various coating agents.

On the other hand, there have been trials to manufacture edible formulations by encapsulating a LAB powder or adding a sugar bead of the LAB powder to various foodstuffs.

However, since the conventional encapsulation of a LAB powder or sugar beads of the lactobacillus powder added to the foodstuffs and medicines are fatal to LAB, the efficiency and effects of LAB in the body cannot be satisfactorily assured.

Hereinafter, the conventional encapsulation and sugar bead-making processes, and problems occurring when the processes are applied to coat LAB, will be explained in detail.

The encapsulation process is first explained. Generally, capsules used to encapsulate foodstuffs and medicines are divided into soft capsule products and hard capsule products. The hard capsule products are manufactured by filling a previously formed capsule with raw materials, while the soft capsule products are manufactured by simultaneously performing both formation of a capsule and filling the capsule with raw materials. Since a polyhydric alcohol as a plasticizer is added to gelatin for manufacturing a soft capsule, the soft capsule is advantageous compared to a hard capsule in terms of flexibility. In addition, the soft capsule can be manufactured in a variety of shapes, such as circular, ovoid, or cylindrical shapes. Furthermore, the soft capsule can be utilized for oral, rectal and vaginal administrations. In particular, when a raw material to be encapsulated is in a liquid state, such as fat, the soft capsule is mainly used.

To manufacture the soft capsule, a rotary die process is widely used. The rotary die process is carried out by feeding an oily material while two gelatin sheets are continuously introduced between two rotating stamping rollers, and compression-molding the gelatin sheets. The specific procedure is as follows:
1) Weighing raw materials,
2) Preparing a material for filling a capsule,
3) Fixing and degassing the material to stabilize it,
4) Forming of a gelatin sheet film,
5) Molding and filling,
6) Drying,
7) Coating.

In step 2), when considering fillability and viscosity, soybean oil, palm hardened oil, lecithin, lead chromate, etc., are mainly used as the material for filling a capsule.

In step 4), gelatin is mainly used as the sheet film. Glycerin and D-sorbitol in a predetermined ratio may be added to the gelatin to determine the hardness of the film. If necessary, a flavoring agent and a coloring agent can be added to the sheet film.

When molding a desired capsule, determination of an appropriate filling amount, molding zone, feeding temperature and solution temperature are necessary. After filling and molding, moisture is removed by drying the molded capsule for 2∼3 days. If necessary, a step of coating the dried capsule with an oil may be carried out to prepare a final product.

Comparing the soft capsule thus prepared with a hard capsule, since the soft capsule restricts the ingress of moisture and air through the film, its shelf life is improved. The improved shelf life greatly contributes to the stability of viable bacteria.

However, when the general method and the raw materials for manufacturing the soft capsule are used to manufacture a soft capsule for encapsulating LAB, there is a problem that most viable cells are killed during molding and drying the soft capsule. Accordingly, the desired objects of a capsule for encapsulating LAB cannot be accomplished. This is because the method and the raw materials for manufacturing the soft capsule are limited only to inanimate objects. That is, the method and raw materials are not suitable for viable bacterial products.

The main causes of death of viable cells during manufacture of a soft capsule for a LAB powder include stress due to the oxidation of oily sub-materials used for the formation of a film and molding the soft capsule, and moisture remaining in the molded capsule. Accordingly, specific raw materials and concentrations of the raw materials capable of appropriately controlling the tensile strength and hardness of the capsule film, and at the same time effectively preventing the death of viable cells, are currently required.

In view of the above-mentioned situations, there is a need for a method for manufacturing a capsule and a composition for manufacturing a capsule film which can manufacture the dosage form of LAB in a capsule form suitable for oral administration, thereby minimizing the death of viable cells during manufacture and maximizing the efficiency of LAB in the body.

Next, as a conventional method for manufacturing a sugar bead, an example of molding a LAB powder in a bead form using alginic acid is known. This method uses the physical properties of alginic acid, such as cross-linkability. According to the method, since an aqueous solution of alginic acid and calcium salt, etc., is used as a cross-linking agent, the applications are highly limited. For this reason, the method of molding a LAB powder in a bead form cannot be applied to the field of foodstuffs. Accordingly, there is a need for a novel method including molding a LAB powder into a spherical bead, coating, coloring and glossing using a common apparatus.

When conventional methods for manufacturing a sugar bead applicable to common foodstuffs are used to encapsulate LAB, most of the LAB die during the procedure. Accordingly, there is a need for a method and a composition capable of easily molding a bead and preventing the death of viable cell, and a composition which can be used in the method.

Other methods exist in the art for extending the shelf life of LABs such as US3,677,897 which describes lactic acid bacteria cultures comprising a lactic acid bacteria cultured in a nutrient medium, an acetylated monoglyceride coating on said culture bacteria and a carrier blended with said coated bacteria, said carrier selected from a group consisting of glucose, modified cellulose and modified starches.

KR2002/069862 describes the manufacturing method for protein coated lactic acid bacteria in which thermostability, acid resistance and bile resistance were increased thereby increasing productivity and reducing production time and cost.

US6,455,052 on the other hand describes the composition for forming an enteric coating on a tablet, capsule or pellet for oral ingestion which includes a liquid mixture of alginic acid particles dispersed in an aqueous solution of a binding agent of locus bean, gum, gelatine, vegetable hydrocolloids and/or animal protein. WO99/20745 also relates to an enteric coated granule prepared by coating lactic acid bacteria containing seed with a water miscible coating material and then, if desired, subjecting the first coated product to a second coating with a controlled release coating material.

### SUMMERY OF THE INVENTION

Therefore, the present invention has been made in view of the above problems, and it is an object of the present invention to provide a double-coated LAB powder which can effectively control the reaction with air and moisture, exhibit excellent resistance to heat, acid and bile, and assure viability, efficiency and effects of LAB.

It is another object of the present invention to provide a method for a method for aseptically preparing the double-coated LAB powder with low-cost materials and low manufacturing costs.

It is yet another object of the present invention to provide a method for manufacturing a dosage form containing the double-coated LAB powder which can manufacture the dosage form in a form suitable for oral administration, and minimize the death of viable cells during manufacturing procedure.

In order to accomplish the above objects of the present invention, there is provided a method for preparing a double-coated LAB powder, comprising the steps of: mixing a predetermined protein to obtain an aqueous protein solution; adding a protease to the aqueous protein solution to enzymatically treat the aqueous protein solution; culturing (fermenting) LAB in the enzymatically treated aqueous protein solution; separating and concentrating the fermented solution using a high-speed centrifugal separator, collecting the LAB, and coating the collected LAB with protein precipitates present in the fermented solution (a first coating step); and homogeneously mixing the coated LAB with an aqueous polysaccharide solution and an aqueous cryoprotectant solution to coat the lactobacillus with the said solution mixture, and lyophilizing the double-coated LAB (a second coating step).

In accordance with one aspect of the present invention, there is provided a method for preparing a double-coated LAB powder, comprising the steps of: mixing a predetermined protein to obtain an aqueous protein solution; adding a protease to the aqueous protein solution to enzymatically treat the aqueous protein solution; culturing (fermenting) LAB in the enzymatically treated aqueous protein solution; separating and concentrating the fermented solution using a high-speed centrifugal separator, collecting the LAB, and coating the collected LAB with protein precipitates present in the fermented solution (a first coating step); mixing an aqueous cryoprotectant solution with the coated LAB and lyophilizing; and homogeneously mixing the lyophilized LAB powder with an aqueous polysaccharide solution to coat the LAB with the polysaccharide solution, and lyophilizing the double-coated LAB (a second coating step).

### BRIEF DESCRIPTION OF THE DRAWINGS

The above and other objects, features and other advantages of the present invention will be more clearly understood from the following detailed description taken in conjunction with the accompanying pictures and drawings, in which:
Picture 1 shows shapes of double-coated LAB powders;
Picture 2 shows a sugar bead containing double-coated Lactic acid bacteria powder;
Picture 3 shows a capsule containing double-coated lactic acid bacteria powder;
Fig. 1 is a process chart showing a conventional method for preparing a coated LAB powder;
Fig. 2 is a process chart showing a method for preparing a LAB powder double-coated using a protein and a polysaccharide, according to one example of the present invention; and
Fig. 3 is a process chart showing a method for preparing a LAB powder double-coated using a protein and a polysaccharide, according to another example of the present invention.

### DESCRIPTION OF THE PREFERRED EMBODIMENTS

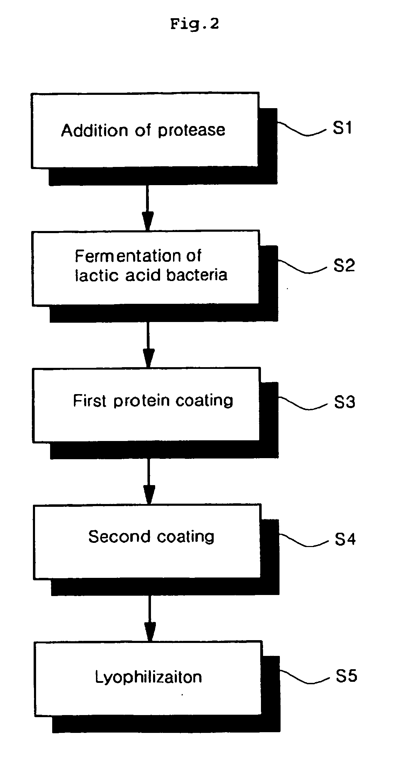

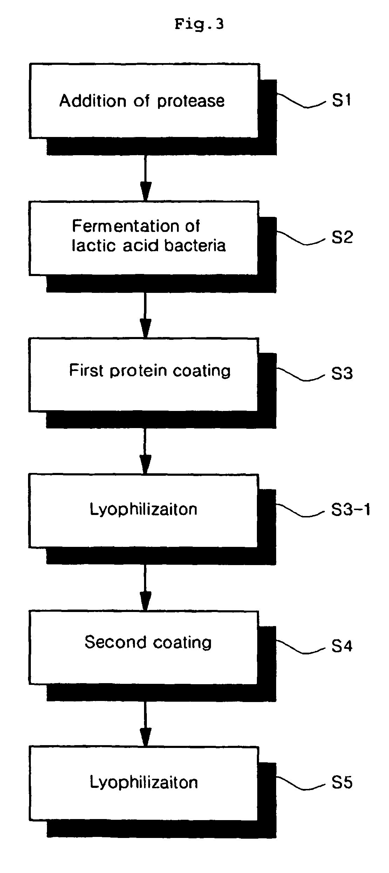

Hereinafter, preferred examples of a method for preparing a LAB powder double-coated using a protein and a polysaccharide according to the present invention will be explained in more detail with reference to the accompanying Figs. 2 and 3.

Fig. 2 shows a process chart of a method for preparing a LAB powder double-coated using a protein and a polysaccharide according to one example of the present invention. Referring to Fig. 2, the collected LAB is doubly coated with a protein and a polysaccharide, and then lyophilized. Fig. 3 shows a process chart of a method for preparing a LAB powder double-coated using a protein and a polysaccharide according to another example of the present invention. Referring to Fig. 3, after the collected LAB is first coated with a protein and lyophilized, the lyophilized LAB powder is doubly coated with an aqueous polysaccharide solution and lyophilized.

According to the methods of the present invention, LAB cells present in a fermentation medium are first coated with a protein-coating agent, and further coated with a mixture of an aqueous polysaccharide solution and an aqueous cryoprotectant solution to prepare a double-coated LAB powder. The methods of the present invention essentially consist of the steps of adding a protease, fermenting LAB, firstly protein coating and secondly coating.

In the step of adding a protease (S1), a 1%-10% aqueous solution of skim milk or isolated soy protein or a mixture thereof is used as a fermentation medium. A protease solution is added to the fermentation medium in an amount of 1∼10% by weight based on the weight of the protein to enzymatically treat the fermentation medium. The amount of the protease added varies according to the kind of LAB species to be coated. The enzymatically-treated hydrolysate of the skim milk and the isolated soy protein includes a water-soluble, low molecular weight peptide ingredient which will be used to proliferate LAB, and a high molecular weight peptide ingredient which is semisoluble in water will be used to coat LAB.

In the LAB fermentation step (S2), after 1~5% by weight of glucose, 0.1∼1.5% by weight of yeast extract, 0.1∼1.5% by weight of a beef extract and 0.01-0.1% by weight of an ionic component selected from ammonium citrate, sodium acetate, dipotassium phosphate, magnesium sulfate, manganese sulfate, sodium chloride, etc., are added to the enzymatically-treated solution and dissolved in the solution, LAB is cultured in a steam-sterilized anaerobic fermentation tube.

In the first protein-coating step (S3), the fermented solution is separated and concentrated using a high-speed centrifugal separator at 15,000rpm or more. At this step, the protein ingredients remaining in the fermented solution are deposited together with the LAB to initiate a coating mechanism. By appropriately controlling the protein concentration and enzymatic treatment ratio of the protease in the hydrolysate composition depending on the kind of LAB species to be coated, the lactobacillus can be rapidly proliferated. In addition, when rapidly frozen, the hydrolysate composition includes the LAB and protects formation of ice crystals, thereby exhibiting a cryoprotecting effect.

In the second coating step (S4), a 35~45% aqueous solution of 1~10% by weight of trehalose, maltodextrin, mannitol and skim milk based on the weight of the collected LAB, is used as a cryoprotectant. After a 1%~10% aqueous solution of 1~10% by weight of xanthan gum, cellulose or levan alone or mixtures thereof based on the weight of the collected LAB is sterilized under pressure, it is mixed with the collected LAB and the cryoprotectant using a stirrer, homogenized, and lyophilized.

According to another example (Fig. 3) of the method for preparing a double-coated LAB powder, a freeze dried bacteria culture powder, which is coated with protein and lyophilized (S3-1), may be mixed into the mixture of the above polysaccharide and cryoprotectant aqueous solution homogeneously using a stirrer and then, freeze-dried whereby the bacteria culture is secondly coated with polysaccharide (S-4).

When the protein-coated LAB uniformly distributed in an aqueous polysaccharide solution is lyophilized, powerful adhesive strength of the polysaccharide helps the formation of clusters having a highly compact structure. The clusters are ground to have a uniform particle size of 40~120 mesh (see Picture 1).

The polysaccharide used as a second coating agent form clusters of LAB due to their powerful adhesive strength. In addition, since the polysaccharide has hydrophilicity, they can be bound with the first coating agent (protein) and can be used in combination with water-soluble sugars and the cryoprotectant (amino acids) and the LAB stabilizer. In particular, the polysaccharide is substantially insoluble in water under acidic conditions and easily dissociated and eluted under neutral or basic conditions. Accordingly, thus treated LAB can survive under stomach acid conditions and become settled in a stable manner in the intestine.

Further, the present invention provides a method for manufacturing a formulation containing the double-coated LAB powder prepared above. In the present invention, the dosage form containing the double-coated LAB powder may be a sugar bead or a soft gelatin capsule.

The method for manufacturing a sugar bead containing the double-coated LAB powder comprises the steps of: spraying an aqueous polysaccharide solution while adding powdered sugar to white sugar under rotation, to obtain a central seed; spraying the aqueous polysaccharide solution while adding a mixture of a double-coated LAB powder and powdered sugar to the central seed under rotation, to mold a sugar bead; and drying the molded sugar bead under aseptic conditions at a temperature of 20°C or less and a relative humidity of 40% or less.

Further, the present invention provides a composition for filling a capsule containing the double-coated lactobacillus powder, comprising a fat-soluble vitamin, a hydrophilic polysaccharide, a vegetable oil, bees-wax, lecithin, palm hardened oil, and the double-coated LAB powder.

Preferably, the composition for filling a capsule comprises 10~50% by weight of a fat-soluble vitamin, 1~30% by weight of a hydrophilic polysaccharide, 15~25% by weight of a vegetable oil, 3~10% by weight of bees-wax, 0.5~3% by weight of lecithin, 10~20% by weight of palm hardened oil, and the balance of the double-coated LAB powder.

As discussed above, the main causes of death of viable cell during manufacture of a capsule for a LAB powder include stress due to the oxidation of oily sub-materials used for the formation of a film and molding a capsule, and moisture remaining in the molded capsule. Since the composition for filling a capsule comprises reduced content of a vegetable oil and further comprises a fat-soluble vitamin, it can minimize stress due to the oily sub-materials. In addition, since the composition for filling a capsule further comprises a hydrophilic polysaccharide, the reaction with moisture can be prevented, thereby greatly contributing to the stability of LAB and minimizing the death of viable cell in the capsule.

A capsule or sugar bead containing the double-coated LAB powder can minimize the death of viable cell during manufacture of the dosage form.

Hereinafter, the method for manufacturing the sugar bead and the composition for filling the capsule are explained, with reference to preferred examples of the method for manufacturing the sugar bead and the capsule.

First, the method for manufacturing the sugar bead is explained in detail. As an apparatus for manufacturing a spherical sugar bead containing the double-coated LAB powder, any apparatuses which can apply a predetermined rotatory force to powder materials, e.g., a coating pan or centrifugal coater, can be used. In addition, polysaccharides, such as sucrose, gum arabic, xanthan gum, cellulose and levan, shellac, various food coloring agents and glossing agents can be used as sub-materials. When the sugar bead is manufactured using a commonly known process, death of LAB may occur. On the contrary, the sugar bead containing the double-coated LAB powder causes no death of viable cell during manufacture. The procedure and raw materials are specifically explained below.

### An example of the method for manufacturing the sugar bead

1) Producing a central seed;
2) Molding a LAB bead;
3) Treating with a flavoring agent and a coloring agent;
4) Treating with a coating agent and a glossing agent;
5) Drying.

**[Table 1]**

| Composition of raw materials for manufacturing a sugar bead | |
|---|---|
| Raw materials | Ratio (% by weight) |
| LAB powder | 3.47 |
| Sucrose | 17.33 |
| Powdered sugar | 77.99 |
| Polysaccharides | 0.52 |
| Flavoring agent | 0.17 |
| Coloring agent | 0.35 |
| Coating agent and glossing agent | 0.17 |

The method for manufacturing the sugar bead is explained in terms of the respective steps. First, an aqueous polysaccharide solution is sprayed while adding powdered sugar to 20~30 mesh sucrose at room temperature under rotation of a coating pan or centrifugal coater, to obtain a central seed.

Next, a mixture of LAB powder and powdered sugar is added to the central seed while spraying the aqueous polysaccharide solution under rotation using a coating pan or centrifugal coater, to mold a sugar bead. At this step, a sweetening agent, a flavoring agent such as fruit-flavored powder, or a coloring agent such as yellow or blue coloring agents, etc., may be mixed with the powdered sugar.

If necessary, a solution containing a coating agent such as Shellac or a glossing agent may be sprayed on the LAB bead, thereby imparting coating and glossing effects to the bead.

Finally, the molded LAB bead is naturally dried in an aseptic room at a temperature of 20°C or less and a relative humidity of 40% or less to manufacture a desired sugar bead. The sugar bead thus manufactured is shown in Picture 2.

As will be apparent from data shown in Test Examples below, the manufactured sugar bead can minimize the death of LAB during the molding step.

Next, the method for manufacturing the soft capsule containing the double-coated LAB powder and the composition for filling the capsule are explained in detail below.

A soft capsule is manufactured by filling a coating agent with a lipophilic filling composition. Since the soft capsule restricts the entrance of moisture and air through the film, its shelf life is improved. The improved shelf life greatly contributes to the stability of viable cell.

On the other hand, the filling composition filled into the soft capsule must have a viscosity and physical properties suitable for the filling, without damaging the stability of a film. For this purpose, the composition is lipophilic and contains a reduced amount of moisture or volatile materials, such as soybean oil, palm hardened oil, lecithin and bees-wax. However, since most microorganisms die in the lipophilic composition, the lipophilic composition cannot be applied to viable bacteria products, such as LAB products.

In summary, the present invention is characterised by a composition for filling a soft capsule applicable to LAB products, without causing any death of viable cells during manufacture of the capsule and damaging the stability of a gelatin film. The method for manufacturing the capsule containing the composition is specifically explained below.
1) Screening of raw materials
In order to prevent the leakage from a soft capsule, a screened powder having a particle size of 80 mesh or more is used as a double-coated lactobacillus powder.
2) Preparation of composition suitable for double-coated LAB powder
- In order to prevent the death of viable cells, the filling composition contains a reduced concentration of a vegetable oil, e.g., soybean oil. Instead, the filling composition further contains a fat-soluble vitamin such as lipophilic squalene in an amount of 10~50% by weight. The fat-soluble vitamin prevents the oxidation of lipids.
- In order to easily pass through a film, exhibit a viscosity sufficient to homogenize and stabilize LAB after drying, hydrophilic polysaccharides such as starches, gums, cellulose and levan are added to the filling composition.
- In order to cause no death of LAB during manufacture of the soft capsule and maintain the stability of lactobacillus during distribution of the soft capsule, the filling composition of the present invention contains ingredients at optimal proportions. A representative example of the optimal proportions is shown in Table 2 below.

**[Table 2] Comparison between conventional filling composition and the filling composition of the present invention**

| conventional filling composition | | Filling composition of the present invention | |
|---|---|---|---|
| Ingredients | Content | Ingredients | Content |
| Powdered raw materials | 5% | Double-coated lactobacillus powder | 5% |
| | - | Corn starch | 10% |
| | - | Squalene | 40% |
| Soybean oil | 74% | Soybean oil | 24% |
| Bee-wax | 5% | Bees-wax | 5% |
| Palm hardened oil | 15% | Palm hardened oil | 15% |
| Lecithin | 1% | Lecithin | 1% |
| Total | 100% | Total | 100% |

3) Defoaming
After mixing, the filling composition having desired properties is depressurized to 70cmHg (700/760atm) to completely remove foams included therein.
4) Formation of gelatin film
As a plasticizer, a mixture of glycerin and D-sorbitol (21.6/8.2 (w/w)) is used. The weight ratio of the plasticizer to gelatin is 0.43: 1. The weight ratio minimizes the occurrence of pores in a film and minimizes the entrance of oxygen and moisture through the film, which is advantageous in terms of the stability of LAB.
5) Molding and drying steps are carried out in accordance with a procedure for manufacturing common soft capsules.

The capsule manufactured in accordance with the method of the present invention as described above is shown in Picture 3.

As will be apparent from data shown in Test Examples below, the manufactured capsule containing the filling composition of the present invention can minimize the death of LAB during manufacture, thereby maximizing the survival rate of LAB included in the capsule.

Hereinafter, the present invention will be described in more detail with reference to the following Examples. However, these Examples are given for the purpose of illustration and are not to be construed as limiting the scope of the invention.

### Example 1: Preparation of double-coated Lactobacillus acidophilus CBT-LH powder

4kg of skim milk and 2kg of isolated soy protein were suspended in 100kg of water. The suspension was placed in an enzyme-treatment bath equipped with a low-speed agitator, a thermostat and a pH-controller, and an aqueous solution of 10.2g of protease-N (Amano) in 100ml of water was added thereto at 55°C and pH 7.0. The mixture was hydrolyzed until the pH decreased to 6.0.

To the hydrolysate, 5kg of glucose, 1kg of a meat extract, 0.5kg of yeast extract, 50g of dipotassium phosphate, 50g of ammonium citrate, 50g of sodium acetate, 10g of magnesium sulfate and 10g of manganese sulfate were added and dissolved. The solution was transferred to a 200L anaerobic fermentation tube, sterilized at 121°C for 15 minutes, and then inoculated with 2L of an inoculum. The resulting solution was fermented for 12 hours while maintaining at a pH of 6.0.

The fermented solution was transferred to a continuous centrifuge at a flow rate of 60L/hr. The LAB and protein remnants were deposited and collected. 100g of xanthan gum, 100g of carboxymethylcellulose and 100g of levan were added to 10L of an aqueous cryoprotectant solution consisting of 1kg of trehalose, 1kg of mannitol and 1kg of maltodextrin, dissolved, sterilized under pressure, homogenized at 5,000 rpm, rapidly frozen in a freezer at -55°C, and lyophilized at a temperature ranging from 0 to 40°C. Protein remnants which are semisoluble in water surrounded the LAB, and water-soluble peptide ingredients were deposited on the cell walls of the LAB, thereby completing a protein coating. Polysaccharide ingredients were formed into clusters having a highly compact structure due to their powerful adhesion to the LAB. The LAB powder double-coated by the mixture of skim milk and isolated soy protein, and the mixture of xanthan gum, carboxymethylcellulose and levan, exhibited improved stability in an accelerated test, acid resistance and bile resistance, compared to an uncoated LAB powder. The results are shown in Table 3 below.

**[Table 3]**

| Results of stability in accelerated test, acid resistance and bile resistance | | | | | | | | |
|---|---|---|---|---|---|---|---|---|
| Acid resistance test (in artificial gastric acid (pH 2.1)) | | | Bile resistance test (in 0.5% Oxgall solution) | | | Accelerated test (40°C,70%(relative humidity)) | | |
| Exposure time (min.) | Test group (cfu/g) | Control group (cfu/g) | Exposure time (min.) | Test group (cfu/g) | Control group (cfu/g) | Time (day) | Test group (cfu/g ) | Control group (cfu/g) |
| 0 | 3.4 x10¹¹ | 3.3 x10¹¹ | 0 | 3.5 x10¹¹ | 3.4 x10¹¹ | 0 | 3.1 x10¹¹ | 3.2 x10¹¹ |
| 30 | 3.2 x10¹¹ | 2.3 x10¹¹ | 30 | 3.3 x10¹¹ | 2.1 x10¹¹ | 10 | 2.9 x10¹¹ | 1.1 x10¹¹ |
| 60 | 3.1 x10¹¹ | 1.5 x10¹¹ | 60 | 3.2 x10¹¹ | 1.4 x10¹¹ | 20 | 2.6 x10¹¹ | 8.9 x10¹⁰ |
| 90 | 3.1 x10¹¹ | 1.2 x10¹¹ | 90 | 3.0 x10¹¹ | 1.1 x10¹¹ | 30 | 2.5 x10¹¹ | 1.0 x10¹⁰ |
| 120 | 2.9 x10¹¹ | 9.3 x10¹⁰ | 120 | 2.9 x10¹¹ | 9.9 x10¹⁰ | 40 | 2.0 x10¹¹ | 9.8 x10⁹ |
| Survival rate after 120 min.(%) | 85.3 | 28.2 | Survival rate after 120 min. (%) | 82.9 | 29.1 | Survival rate after 40 days(%) | 64.5 | 3.1 |

### Example 2: Manufacture of spherical bead containing double-coated LAB powder

10kg of a sucrose powder having a particle size of 20~30 mesh was charged into a centrifugal coater at 100rpm, and 15kg of powdered sugar was slowly added thereto while spraying 15% aqueous gum arabic solution, to produce a central seed. 2kg of a double coated LAB powder and 10kg of powdered sugar were mixed with the central seed, and then moulded into a first spherical bead in the same manner as described above.

10kg of powdered sugar was added to the molded spherical bead in the same manner as described above, to form a film. 10kg of powdered sugar, 0.1kg of a lemon flavored powder and 0.2kg of a gardenia yellow colorant were mixed with the film to carry out sweetening, flavoring and coloring steps in the same manner as described above. The film was coated and glossed by spraying a 0.7% aqueous shellac solution, and then naturally dried in a closed clean room at 18°C and a relative humidity of 40% until the moisture content reached 4% or less.

Respective ingredients used to manufacture the sugar bead in this Example and contents thereof are shown in Table 4 below. The survival rates of LAB in the sugar bead are shown in Table 5 below.

**[Table 4]**

| Ingredients for manufacture of LAB bead and contents thereof. | | |
|---|---|---|
| Raw materials | | Weight (Kg) |
| LAB powder | Double-coated LAB (viable cell counts 3x10¹¹/g) | 2 |
| Sucrose | Sucrose (20-30 mesh) | 10 |
| Powdered sugar For caking prevention | Powdered sugar | 45 |
| Polysaccharide For adhesion | 15% aqueous arabic gum solution | 0.3 |
| Flavoring agent | Lemon flavored powder | 0.1 |
| Coloring agent | Gardenia yellow colorant | 0.2 |
| Coating agent and glossing agent | Shellac | 0.1 |
| Total weight | | 57.7 |

**[Table 5]:**

| Comparison between survival rates of LAB in sugar bead of the present invention and conventional sugar bead | | | |
|---|---|---|---|
| Conventional sugar bead (cfu/g) | | Sugar bead of the present invention (cfu/g) | |
| (L.acidophillus) | | (L.acidophillus) | |
| Before molding | 6.0×10⁹ | Before molding | 6.9×10⁹ |
| After molding | 9.4×10⁸ | After molding | 4.3×10⁹ |
| After drying | 2.3×10⁸ | After drying | 1.4×10⁹ |

### Example 3: Manufacture of soft capsule using double-coated LAB powder

A mixture of 40kg of squalene, 24kg of soybean oil, 5kg of bee-wax, 15kg of palm hardened oil and 1kg of lecithin was stirred using an agi-homomixer at 75rpm and 62°C, and cooled with continuous stirring. To the solution, 5kg of a double-coated LAB powder having a particle size of 80 mesh or more and 10kg of corn starch were added. The resulting mixture was homogeneously stirred using an agi-homomixer at 78rpm and room temperature, and completely defoamed at reduced pressure of 70cmHg (700/760atm,0.092 Mpa).

68.8kg of gelatin, 21.6kg of glycerin, 8.2kg of D-sorbitol, 0.16kg of ethyl vanillin, 0.69kg of titanium dioxide, and food coloring agents (Blue #1 and Yellow #4) added to 63.4kg of purified water to form a film having a thickness of 0.5-0.8mm. The film was molded into a soft capsule having an adhesive thickness of 0.175mm or more. The molded soft capsule was subjected to a tumble-drying at 16~22Hz for 2~3 hours, and then further dried in a drying room for 2~3 days, to manufacture a LAB soft capsule. After drying, moisture contents on and within the film were 7% and 10% or less, respectively.

Ingredients, contents thereof and process conditions for manufacturing the soft capsule in this Example are shown in Table 6 below. The survival rates of LAB in the capsule are shown in Table 7 below.

**[Table 6]:**

| Ingredients for manufacture of capsule and contents thereof | | | | | | | | | | | |
|---|---|---|---|---|---|---|---|---|---|---|---|
| Procedure | Ingredients and contents thereof | | | | | | | | | | |
| Preparation of filling composition | Ingredients | | | | | | Contents (%) | | | | |
| | Double-coated LAB powder | | | | | | 5 | | | | |
| | Corn starch | | | | | | 10 | | | | |
| | Squalene | | | | | | 40 | | | | |
| | Soybean oil | | | | | | 24 | | | | |
| | Bee-wax | | | | | | 5 | | | | |
| | Palm hardened oil | | | | | | 15 | | | | |
| | Lecithin | | | | | | 1 | | | | |

| Formation of soft capsule film | Ingredients | | | | | | Contents (%) | | | | |
|---|---|---|---|---|---|---|---|---|---|---|---|
| | Distilled water | | | | | | 74.770 of gelatin | | | | |
| | Gelatin | | | | | | 68.850 | | | | |
| | Glycerin | | | | | | 21.629 | | | | |
| | D-sorbitol | | | | | | 8.197 | | | | |
| | Ethyl vanillin | | | | | | 0.164 | | | | |
| | Titanium dioxide | | | | | | 0.689 | | | | |
| | Food coloring agent Blue #1 | | | | | | 0.069 | | | | |
| | Food coloring agent Yellow #4 | | | | | | 0.413 | | | | |
| Molding | Spec. | Die | Molding speed | | Material | Film | Segment | Drum | | Film thickness | Temp. of product |
| | Values | Oval 6 | 2~3rpm | | 21~ 25°C | 50°C | 35~ 50°C | 15~2 5°C | | 0.5~0. 8mm | 25~28°C |
| Drying | Spec. | Tumble-drying | | Tumbling speed | | Drying in drying room | Moisture content on film | | Moisture content within film | | |
| | Values | 2~3 hours | | 16-22Hz | | 2~3 days | 7% | | 10% or less | | |

**[Table 7] :**

| Comparison between survival rates of LAB in capsule of the present invention and conventional capsule | | | |
|---|---|---|---|
| Type A (Conventional capsule) | | Type B (Capsule of the present invention) | |
| Double-coated LAB powder | 5% | Double-coated LAB powder | 5% |
| | | Corn starch | 10% |
| | | Squalene | 40% |
| Soybean oil | 74% | Soybean oil | 24% |
| Bee-wax | 5% | Bees-wax | 5% |
| Palm hardened oil | 15% | Palm hardened oil | 15% |
| Lecithin | 1% | Lecithin | 1% |
| Total | 100% | Total | 100% |
| Viscosity | | Viscosity | |
| Specific gravity | | Specific gravity | |
| pH | | pH | |
| Survival rates of LAB during manufacture (cfu/g) | | | |
| Before molding | 9.4×10⁹ | Before molding | 9.1×10⁹ |
| After molding, before drying | 3.0×10⁹ | After molding, before drying | 8.8×10⁹ |
| After drying | 6.0×10⁸ | After drying | 6.8×10⁹ |

According to the method for preparing the double-coated LAB powder of the present invention, the combination with various cryoprotectants, stabilizers, etc., is possible depending on the kind of LAB species to be coated. Since the double-coated LAB powder can be easily prepared through homogenization and lyophilization steps, the method can be carried out without any additional facilities, and can be applied to centrifuged cell pellets or freeze-dried LAB powders. Accordingly, the method has advantages of excellent adaptability and compatibility. In addition, since the collection rate of the LAB powder can be increased to 50~90% using the method, high productivity is accomplished. The double-coated LAB powder thus prepared is advantageous in terms of excellent resistance to heat, acid and bile, and improved shelf-life. Furthermore, when the double-coated LAB powder is administered to the human body, it can be stably settled in the intestine and thus sufficiently exerts physiological activities of LAB, which maximizes the usability of the double-coated LAB powder.

Although the preferred embodiments of the present invention have been disclosed for illustrative purposes, those skilled in the art will appreciate that various modifications, additions and substitutions are possible, without departing from the scope of the invention as claimed in the accompanying claims.

## Claims

1. A method for preparing a double-coated Lactic acid bacteria powder, comprising the steps of:
mixing a predetermined protein to obtain an aqueous protein solution;
adding a protease to the aqueous protein solution to enzymatically treat the aqueous protein solution;
fermenting Lactic acid bacteria in the enzymatically treated aqueous protein solution;
separating and concentrating the fermented solution using a high-speed centrifugal separator wherein the lactic acid bacteria is coated with protein precipitates present in the fermented solution (a first coating step); and
homogeneously mixing the coated Lactic acid bacteria witch a mixture of an aqueous polysaccharide solution and an aqueous cryoprotectant solution to coat the Lactic acid bacteria with said solution mixture, and lyophilizing the double-coated Lactic acid bacteria (a second coating step).

2. A method according to claim 1 wherein the polysaccharide provided is selected from the group consisting of xanthan gum, cellulose, levan and mixtures thereof.

3. A method according to claims 1 or 2 wherein the protein provided is selected from the group consisting of skim milk, isolated soy protein and a mixture thereof.

4. A method according to any one of claims 1-3 wherein the method further comprises the step of mixing glucose, yeast extract, beef extract and an ionic component with the aqueous protein solution, prior to the fermenting step.

5. The method according to any one of claims 1-4 wherein the ionic component provided is selected from ammonium citrate, sodium acetate, dipotassium phosphate, magnesium sulfate, manganese sulfate and sodium chloride and mixtures thereof.

6. A method for preparing a double-coated Lactic acid bacteria powder, comprising the steps of:
mixing a predetermined protein to obtain an aqueous protein solution;
adding a protease to the aqueous protein solution to enzymatically treat the aqueous protein solution;
culturing (fermenting) Lactic acid bacteria in the enzymatically treated aqueous protein solution;
separating and concentrating the fermented solution using a high-speed centrifugal separator wherein the Lactic acid bacteria is coated with protein precipitates present in the fermented solution (a first coating step);
mixing an aqueous cryoprotectant solution with the protein coated Lactic acid bacteria, and lyophilizing; and
homogeneously mixing the said lyophilized Lactic acid bacteria powder with an aqueous polysaccharide solution such as to coat the Lactic acid bacteria with a mixture of the aqueous cryoprotectant solution and the aqueous polysaccharide solution, and lyophilizing the resultant double-coated Lactic acid bacteria (a second coating step).

7. A double-coated Lactic acid bacteria powder prepared in accordance with any one of claims 1 to 6.

8. A method for manufacturing a sugar bead containing a double-coated Lactic acid bacteria powder prepared in accordance with the method of any one of claims 1 to 6, comprising the steps of:
spraying an aqueous polysaccharide solution while adding powdered sugar to sucrose under rotation, to obtain a central seed;
spraying the aqueous polysaccharide solution while adding a mixture of a double-coated Lactic acid bacteria powder and powdered sugar to the central seed under rotation, to mold a sugar bead; and
drying the molded sugar bead under aseptic conditions at a temperature of 20°C or less and a relative humidity of 40% or less.

9. A method according to claim 8 wherein a sweetening agent, a flavoring agent or a coloring agent is provided and mixed in during the molding step.

10. A method according to claim 8 wherein a coating agent or glossing agent is sprayed onto the molded sugar bead, following the molding step.

11. A method according to any one of claims 8-10 wherein the polysaccharide provided is selected from the group consisting of xanthan gum, cellulose, levan and mixtures thereof.

12. A composition for filling a soft capsule containing a double-coated Lactic acid bacteria powder, comprising a fat-soluble vitamin, a hydrophilic polysaccharide, a vegetable oil, bees-wax, lecithin, palm hardened oil, and a double-coated Lactic acid bacteria powder prepared in accordance with the method of any one of claims 1 to 6.

13. A composition according to claim 12 wherein the composition comprises 10~50% by weight of the fat-soluble vitamin, 1~30% by weight of the hydrophilic polysaccharide, 15~25% by weight of the vegetable oil, 3~10% by weight of bee-wax, 0.5~3% by weight of lecithin, 10~20% by weight of palm hardened oil, and the balance of the double-coated Lactic acid bacteria powder.

14. The composition according to claims 12 or 13 wherein the fat-soluble vitamin is squalene.

15. The composition according to claims 12-14 wherein the hydrophilic polysaccharide is selected from the group consisting of starches, gums, cellulose, levan and mixtures thereof.

## Patentansprüche

1. Ein Verfahren zum Herstellen eines Pulvers aus doppelt beschichteten Milchsäurebakterien, das die folgenden Schritte beinhaltet:
Mischen eines vorher festgelegten Proteins, um eine wässrige Proteinlösung zu erhalten;
Hinzufügen einer Protease zu der wässrigen Proteinlösung, um die wässrige Proteinlösung enzymatisch zu behandeln;
Fermentieren der Milchsäurebakterien in der enzymatisch behandelten wässrigen Proteinlösung;
Abscheiden und Konzentrieren der fermentierten Lösung unter der Verwendung eines Zentrifugalabscheiders hoher Geschwindigkeit, wobei die Milchsäurebakterien mit Proteinniederschlägen, die in der fermentierten Lösung vorhanden sind, beschichtet werden (ein erster Beschichtungsschritt); und
homogenes Mischen der beschichteten Milchsäurebakterien mit einer Mischung aus einer wässrigen Polysaccharidlösung und einer wässrigen Kryoschutzmittellösung, um die Milchsäurebakterien mit der Lösungsmischung zu beschichten, und Lyophilisieren der doppelt beschichteten Milchsäurebakterien (ein zweiter Beschichtungsschritt).

2. Verfahren gemäß Anspruch 1, wobei das bereitgestellte Polysaccharid aus der Gruppe, bestehend aus Xanthan, Cellulose, Levan und Mischungen daraus, ausgewählt wird.

3. Verfahren gemäß Anspruch 1 oder 2, wobei das bereitgestellte Protein aus der Gruppe, bestehend aus Magermilch, isoliertem Sojaprotein und einer Mischung daraus, ausgewählt wird.

4. Verfahren gemäß einem der Ansprüche 1-3, wobei das Verfahren des Weiteren den Schritt des Mischens von Glucose, Hefeextrakt, Rinderextrakt und einer ionischen Komponente mit der wässrigen Proteinlösung vor dem Fermentierungsschritt beinhaltet.

5. Verfahren gemäß einem der Ansprüche 1-4, wobei die bereitgestellte ionische Komponente aus Ammoniumcitrat, Natriumacetat, Dikaliumphosphat, Magnesiumsulfat, Mangansulfat und Natriumchlorid und Mischungen daraus ausgewählt wird.

6. Ein Verfahren zum Herstellen eines Pulvers aus doppelt beschichteten Milchsäurebakterien, das die folgenden Schritte beinhaltet:
Mischen eines vorher festgelegten Proteins, um eine wässrige Proteinlösung zu erhalten;
Hinzufügen einer Protease zu der wässrigen Proteinlösung, um die wässrige Proteinlösung enzymatisch zu behandeln;
Züchten (Fermentieren) der Milchsäurebakterien in der enzymatisch behandelten wässrigen Proteinlösung;
Abscheiden und Konzentrieren der fermentierten Lösung unter der Verwendung eines Zentrifugalabscheiders hoher Geschwindigkeit, wobei die Milchsäurebakterien mit Proteinniederschlägen, die in der fermentierten Lösung vorhanden sind, beschichtet werden (ein erster Beschichtungsschritt);
Mischen einer wässrigen Kryoschutzmittellösung mit den mit Protein beschichteten Milchsäurebakterien und Lyophilisieren; und
homogenes Mischen des Pulvers aus lyophilisierten Milchsäurebakterien mit einer wässrigen Polysaccharidlösung, um die Milchsäurebakterien mit einer Mischung aus der wässrigen Kryoschutzmittellösung und der wässrigen Polysaccharid lösung zu beschichten, und Lyophilisieren der resultierenden doppelt beschichteten Milchsäurebakterien (ein zweiter Beschichtungsschritt).

7. Ein Pulver aus doppelt beschichteten Milchsäurebakterien, das nach einem der Ansprüche 1 bis 6 hergestellt wurde.

8. Ein Verfahren zum Erzeugen eines Zuckerkügelchens, das ein Pulver aus doppelt beschichteten Milchsäurebakterien enthält, das nach dem Verfahren gemäß einem der Ansprüche 1 bis 6 hergestellt wurde, das die folgenden Schritte beinhaltet:
Sprühen einer wässrigen Polysaccharidlösung während des Hinzufügens von pulverförmigem Zucker zu Saccharose unter Rotation, um einen Zentralkeim zu erhalten;
Sprühen der wässrigen Polysaccharidlösung während des Hinzufügens einer Mischung aus einem Pulver aus doppelt beschichteten Milchsäurebakterien und pulverförmigem Zucker zu dem Zentralkeim unter Rotation, um ein Zuckerkügelchen zu formen; und
Trocknen des geformten Zuckerkügelchens unter aseptischen Bedingungen bei einer Temperatur von 20 °C oder weniger und einer relativen Feuchtigkeit von 40 % oder weniger.

9. Verfahren gemäß Anspruch 8, wobei ein Süßungsmittel, ein Aromastoff oder ein Farbstoff bereitgestellt und während des Formungsschritts beigemischt wird.

10. Verfahren gemäß Anspruch 8, wobei im Anschluss an den Formungsschritt ein Beschichtungsstoff oder ein Glanzstoff auf das geformte Zuckerkügelchen gesprüht wird.

11. Verfahren gemäß einem der Ansprüche 8-10, wobei das bereitgestellte Polysaccharid aus der Gruppe, bestehend aus Xanthan, Cellulose, Levan und Mischungen daraus, ausgewählt wird.

12. Eine Zusammensetzung zum Füllen einer Weichkapsel, die ein Pulver aus doppelt beschichteten Milchsäurebakterien enthält, die ein fettlösliches Vitamin, ein hydrophiles Polysaccharid, ein pflanzliches Öl, Bienenwachs, Lecithin, gehärtetes Palmöl und ein Pulver aus doppelt beschichteten Milchsäurebakterien, das nach dem Verfahren gemäß einem der Ansprüche 1 bis 6 hergestellt wurde, beinhaltet.

13. Zusammensetzung gemäß Anspruch 12, wobei die Zusammensetzung 10~50 Massenanteile in % des fettlöslichen Vitamins, 1~30 Massenanteile in % des hydrophilen Polysaccharids, 15~25 Massenanteile in % des pflanzlichen Öls, 3~10 Massenanteile in % des Bienenwachs, 0,5~3 Massenanteile in % des Lecithins, 10~20 Massenanteile in % des gehärteten Palmöls und für den Rest das Pulver aus doppelt beschichteten Milchsäurebakterien beinhaltet.

14. Zusammensetzung gemäß Anspruch 12 oder 13, wobei das fettlösliche Vitamin Squalen ist.

15. Zusammensetzung gemäß Ansprüchen 12-14, wobei das hydrophile Polysaccharid aus der Gruppe, bestehend aus Stärken, Gummis, Cellulose, Levan und Mischungen daraus, ausgewählt wird.

## Revendications

1. Une méthode destinée à la préparation d'une poudre de bactéries lactiques à double enrobage, comprenant les étapes de :
mélanger une protéine prédéterminée pour obtenir une solution de protéine aqueuse ;
ajouter une protéase à la solution de protéine aqueuse pour traiter de façon enzymatique la solution de protéine aqueuse ;
fermenter les bactéries lactiques dans la solution de protéine aqueuse traitée de façon enzymatique ;
séparer et concentrer la solution fermentée en utilisant un séparateur centrifuge à vitesse élevée dans lequel les bactéries lactiques sont enrobées de précipités de protéine présents dans la solution fermentée (une première étape d'enrobage) ; et
mélanger de façon homogène les bactéries lactiques enrobées avec un mélange d'une solution de polysaccharide aqueuse et d'une solution cryoprotectrice aqueuse pour enrober les bactéries lactiques dudit mélange de solutions, et lyophiliser les bactéries lactiques à double enrobage (une deuxième étape d'enrobage).

2. Une méthode selon la revendication 1 dans laquelle le polysaccharide fourni est sélectionné dans le groupe consistant en gomme de xanthane, cellulose, lévane et des mélanges de ceux-ci.

3. Une méthode selon les revendications 1 ou 2 dans laquelle la protéine fournie est sélectionnée dans le groupe consistant en lait écrémé, protéine de soja isolée et un mélange de ceux-ci.

4. Une méthode selon n'importe laquelle des revendications 1 à 3 dans laquelle la méthode comprend de plus l'étape de mélanger du glucose, de l'extrait de levure, de l'extrait de boeuf et un composé ionique avec la solution de protéine aqueuse, avant l'étape de fermentation.

5. La méthode selon n'importe laquelle des revendications 1 à 4 dans laquelle le composé ionique fourni est sélectionné parmi le citrate d'ammonium, l'acétate de sodium, le phosphate dipotassique, le sulfate de magnésium, le sulfate de manganèse et le chlorure de sodium et des mélanges de ceux-ci.

6. Une méthode destinée à la préparation d'une poudre de bactéries lactiques à double enrobage, comprenant les étapes de :
mélanger une protéine prédéterminée pour obtenir une solution de protéine aqueuse ;
ajouter une protéase à la solution de protéine aqueuse pour traiter de façon enzymatique la solution de protéine aqueuse ; cultiver (fermenter) les bactéries lactiques dans la solution de protéine aqueuse traitée de façon enzymatique ;
séparer et concentrer la solution fermentée en utilisant un séparateur centrifuge à vitesse élevée dans lequel les bactéries lactiques sont enrobées de précipités de protéine présents dans la solution fermentée (une première étape d'enrobage) ;
mélanger une solution cryoprotectrice aqueuse avec les bactéries lactiques enrobées de protéine, et lyophiliser ; et
mélanger de façon homogène ladite poudre de bactéries lactiques lyophilisées avec une solution de polysaccharide aqueuse de façon à enrober les bactéries lactiques d'un mélange de la solution cryoprotectrice aqueuse et de la solution de polysaccharide aqueuse, et lyophiliser les bactéries lactiques à double enrobage résultantes (une deuxième étape d'enrobage).

7. Une poudre de bactéries lactiques à double enrobage préparée conformément à n'importe laquelle des revendications 1 à 6.

8. Une méthode destinée à la fabrication d'une perle de sucre contenant une poudre de bactéries lactiques à double enrobage préparée conformément à la méthode de n'importe laquelle des revendications 1 à 6, comprenant les étapes de :
vaporiser une solution de polysaccharide aqueuse tout en ajoutant du sucre en poudre à de la sucrose en rotation, afin d'obtenir un noyau central ;
vaporiser la solution de polysaccharide aqueuse tout en ajoutant un mélange d'une poudre de bactéries lactiques à double enrobage et de sucre en poudre au noyau central en rotation, afin de former une perle de sucre ; et
sécher la perle de sucre formée en conditions d'asepsie à une température de 20 °C ou inférieure et une humidité relative de 40 % ou inférieure.

9. Une méthode selon la revendication 8 dans laquelle un agent édulcorant, un aromatisant ou un colorant est fourni et incorporé lors de l'étape de formation.

10. Une méthode selon la revendication 8 dans laquelle un agent d'enrobage ou un agent de glaçage est vaporisé sur la perle de sucre formée, suivant l'étape de formation.

11. Une méthode selon n'importe laquelle des revendications 8 à 10 dans laquelle le polysaccharide fourni est sélectionné dans le groupe consistant en gomme de xanthane, cellulose, lévane et des mélanges de ceux-ci.

12. Une composition destinée au remplissage d'une gélule molle contenant une poudre de bactéries lactiques à double enrobage, comprenant une vitamine liposoluble, un polysaccharide hydrophile, une huile végétale, de la cire d'abeilles, de la lécithine, de l'huile de palme durcie, et une poudre de bactéries lactiques à double enrobage préparée conformément à la méthode de n'importe laquelle des revendications 1 à 6.

13. Une composition selon la revendication 12 dans laquelle la composition comprend de 10 à 50 % en poids de vitamine liposoluble, de 1 à 30 % en poids de polysaccharide hydrophile, de 15 à 25 % en poids d'huile végétale, de 3 à 10 % en poids de cire d'abeilles, de 0,5 à 3 % en poids de lécithine, de 10 à 20 % en poids d'huile de palme durcie, et le solde de poudre de bactéries lactiques à double enrobage.

14. La composition selon les revendications 12 ou 13 dans laquelle la vitamine liposoluble est le squalène.

15. La composition selon les revendications 12 à 14 dans laquelle le polysaccharide hydrophile est sélectionné dans le groupe consistant en amidons, gommes, cellulose, lévane et des mélanges de ceux-ci.
